(19) Europäisches Patentamt
European Patent Office
Office européen des brevets

(11) Veröffentlichungsnummer: **0 295 578**
**B1**

(12) # EUROPÄISCHE PATENTSCHRIFT

(45) Veröffentlichungstag der Patentschrift: **05.12.90**

(51) Int. Cl.[5]: **B01J 31/04,** C07C 41/03

(21) Anmeldenummer: **88109241.5**

(22) Anmeldetag: **10.06.88**

(54) **Verwendung von Erdalkalisalzen von Ethercarbonsäuren als Katalysatoren für die Alkoxylierung.**

(30) Priorität: **15.06.87 DE 3719968**
**25.01.88 DE 3802044**

(43) Veröffentlichungstag der Anmeldung:
**21.12.88 Patentblatt 88/51**

(45) Bekanntmachung des Hinweises auf die Patenterteilung:
**05.12.90 Patentblatt 90/49**

(84) Benannte Vertragsstaaten:
**AT BE CH DE ES FR GB GR IT LI LU NL SE**

(56) Entgegenhaltungen:
**EP-A- 0 082 554**
**EP-A- 0 082 569**
**US-A- 3 917 676**

(73) Patentinhaber: **Henkel Kommanditgesellschaft auf Aktien, Postfach 1100 Henkelstrasse 67, D-4000 Düsseldorf-Holthausen(DE)**

(72) Erfinder: **Behler, Ansgar, Dr., Wittekindstrasse 44, D-4250 Bottrop(DE)**
Erfinder: **Ploog, Uwe, Dr., Haydenweg 6, D-5657 Haan(DE)**
Erfinder: **Scholz, Elvira, Werstener Dorfstrasse 106, D-4000 Düsseldorf 13(DE)**

**EP 0 295 578 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft die Verwendung von Erdalkalisalzen von Ethercarbonsäuren der allgemeinen Formel I

$[RO\text{-}(C_mH_{2m}O)_n\text{-}CH_2\text{-}COO^-]_x\ M^{2+}$ (I)

in der
R ein Rest aus der von $C_1$ bis $C_{22}$-Alkyl, $C_3$ bis $C_{22}$-Alkenyl, Phenyl, Alkylphenyl mit 1 bis 3 $C_1$ bis $C_{15}$-Alkylresten, Benzyl und Phenylethyl gebildeten Gruppe,
M ein Erdalkalimetall aus der von Ca, Sr und Ba gebildeten Gruppe,
m die Zahl 2 und/oder 3 und
n die Zahl 1 bis 20 und
x die Zahl 2
oder
R eine Gruppe der Formel

$-CH_2\text{-}COO^-$

x die Zahl 1
und
M, m und n wie oben definiert sind,
als Katalysatoren für die Ethoxylierung und/oder Propoxylierung von organischen Verbindungen mit aktiven H-Atomen.

Unter organischen Verbindungen mit aktiven H-Atomen werden im Zusammenhang mit der Erfindung solche organischen Verbindungen verstanden, die im Molekül mindestens ein H-Atom enthalten, das in der Lage ist, mit Ethylenoxid und/oder Propylenoxid zu reagieren. Organische Verbindungen mit aktiven H-Atomen sind insbesondere aliphatische Monoalkohole, aliphatische Polyole, aliphatische und aromatische Carbonsäuren, Partialester aus aliphatischen Polyolen und aliphatischen oder aromatischen Carbonsäuren, Hydroxycarbonsäuren, Hydroxycarbonsäureester, Phenol und Alkylphenole, aliphatische und aromatische Amine sowie Amide und Alkylolamide von aliphatischen oder aromatischen Carbonsäuren. Aus den genannten organischen Verbindungen mit aktiven H-Atomen entstehen bei der Umsetzung mit Ethylenoxid und/oder Propylenoxid nichtionogene oberflächenaktive Substanzen, die unter anderem als Detergentien praktische Verwendung finden.

Ein typisches Beispiel für die Reaktion von Ethylenoxid und/oder Propylenoxid mit organischen Verbindung mit aktiven H-Atomen ist die Umsetzung von Fettalkoholen mit üblicherweise 10 bis 18 Kohlenstoffatomen mit Ethylenoxid und/oder Propylenoxid in Gegenwart von Katalysatoren, wobei die Fettalkohole mit mehreren Molekülen Ethylenoxid und/oder Propylenoxid reagieren.

Als Katalysatoren für die vorgenannte Polyalkoxylierung sind hierfür u. a. die folgenden eingesetzt worden:
Calcium- und Strontiumhydroxid, -alkoxide und phenoxide (EP-A 00 92 256),
Calciumalkoxide (EP-A 00 91 146),
Bariumhydroxid (EP-B 01 15 083),
basische Magnesiumverbindungen, z. B. Alkoxide (EP-A 00 82 569),
Magnesium- und Calciumfettsäuresalze (EP-A 0 85 167).

Die vorgenannen Katalysatoren weisen u.a. den Nachteil auf, daß sie schlecht in das Reaktionssystem einarbeitbar und/oder schwierig herstellbar sind.

Gebräuchliche Polyalkoxylierungskatalysatoren sind weiterhin Kaliumhydroxid und Natriummethylat.

Für Fettalkoholpolyalkoxylate ist eine enge Bandbreite des Polyalkoxylierungsgrades von besonderer Bedeutung, vgl. JAOCS, Vol. 63, 691 - 695 (1986), und HAPPI, 5 (1968) S. 52, 54, 123. die sogenannten "narrow-range"-Alkoxylate weisen demnach insbesondere die folgenden Vorteile auf:
- niedrige Fließpunkte
- höhere Rauchpunkte
- weniger Mole Alkoxid zum Erreichen der Wasserlöslichkeit
- weniger Hydrotope für das Einbringen in flüssige Universalwaschmittel
- ein geringerer, durch Anwesenheit freier (nicht umgesetzter) Fettalkohole bedingter Geruch
- Reduzierung des Plumings beim Sprühtrocknen von Waschmittelslurries, die Fettalkoholpolyalkoxylat-Tenside enthalten.

Die Bandbreite bzw. Homologenverteilung der Fettalkoholpolyalkoxylate hängt wesentlich von der Art des verwendeten Katalysators ab. Als Maß für die Homologenverteilung gilt der sogenannte Q-Wert gemäß der Beziehung
$Q = \bar{n} \cdot p^2$
in der $\bar{n}$ die durchschnittliche Adduktzahl (mittlerer Ethoxylierungsgrad) und p den Prozentsatz des Adduktes mit bestimmten EO-Grad, das überwiegend gebildet wird, bedeuten. Ein großer Q-Wert bedeutet somit eine enge Bandbreite der Homologenverteilung.

Die den erfindungsgemäß zu verwendenden Erdalkalisalzen zugrunde liegenden Ethercarbonsäuren sind bekannte Verbindungen, die im Handel erhältlich sind und z. B. nach der DE-B 24 18 444 hergestellt werden können. Alkali- und Erdalkalisalze dieser Ethercarbonsäuren sind als solche ebenfalls bekannt und sind als hautschonende, gut schäumende Tenside eingesetzt wor den, z. B. Körperpflegemitteln (JP-A 86/21 199), in kosmetischen und pharmazeutischen Formulierungen (DE-A 3 521 505, JP-A 82/202 391), in Reinigungsformulierungen (JP-A 85/185 298, BE-A 888 761) sowie als geschmacklose Tenside in Zahncremes (US-A 4 130 636, GB-A 1 526 379), weiterhin in Polstermöbel-Shampoos (DE-A 2 155 004) sowie als Tenside in der tertiären Erdölförderung (DE-A 3 307 712, EP-A 73 894, EP-A 47 370).

Es wurde nun gefunden, daß man unter erfindungsgemäßer Verwendung der Erdalkalisalze von Ethercarbonsäuren der obigen Formel I ebenfalls zu polyethoxylierten und/oder polypropoxylierten Derivaten von organischen Verbindungen mit aktiven H-Atomen mit einer engen Bandbreite der Homologenverteilung kommt, insbesondere zu polyethoxylierten und/oder polypropoxylierten Fettalkoholen mit hohem Q-Wert. Gegenüber den vorbekannten Katalysatoren weisen die erfindungsgemäß zu verwendenden Erdalkalisalze den Vorteil auf, daß sie kurze Reaktionszeiten ermöglichen und infolge ihrer Alkhollöslichkeit im Reaktionssystem, anderes als z. B. Erdalkalisalze gemäß dem Stand der Technik, löslich sind.

Die den erfindungsgemäß zu verwendenden Erdalkalisalzen zugrunde liegenden Ethercarbonsäuren der allgemeinen Formel I leiten sich formal ab von einer polyalkoxylierten α-Hydroxyessigsäure, wobei die Polyalkoxylgruppen von Ethylenoxid oder Propylenoxid oder von Ethylenoxid und Propylenoxid in random- oder Block-Verteilung gebildet sind; im Falle der Bildung mit Propylenoxid läßt sich die Position der Methylgruppen nicht genau angeben. Bevorzugt werden Ethercarbonsäuren, deren Polyalkoxykette ausschließlich von Ethylenoxid gebildet ist (m = 2). Die freie Hydroxylgruppe der Polyalkoxykette ist mit einem Alkohol oder Phenol der Formel ROH verethert, wobei der Rest R aus der von geradkettigem oder verzweigtem $C_1$ bis $C_{22}$-Alkyl, geradkettigem oder verzweigtem $C_3$ bis $C_{22}$-Alkenyl, Phenyl, Alkylphenyl mit 1 bis 3 $C_1$ bis $C_{15}$-Alkylresten, Benzyl und Phenylethyl gebildeten Gruppe ausgewählt ist. Bevorzugt sind Erdalkalisalze von Ethercarbonsäuren, bei denen die die Gruppe R eine $C_1$-$C_8$-Alkylgruppe ist, weil diese Verbindungen in Methanol löslich sind. Die höher alkylierten Derivate sind lediglich in höheren Alkoholen als Methanol löslich, können jedoch ohne weiteres auch als Alkoxylierungskatalysatoren im Sinne der Erfindung eingesetzt werden.

Bevorzugt ist weiterhin die Verwendung von Erdalkalisalzen von Ethercarbonsäuren der allgemeinen Formel 1, in der n eine Zahl von 1 bis 12 bedeutet; Verbindungen, in denen n größer als 20 wird, sind weniger interessant, weil ihre katalytische Aktivität sich vom Optimum entfernt.

Die zur Verwendung im Rahmen der Erfindung vorgesehenen Calcium-, Strontium- und Bariumseifen können nach den für die Herstellung von Erdalkaliseifen von Carbonsäuren bekannten Verfahren erhalten werden.

Die Ethoxylierung und/oder Propoxylierung von organischen Verbindungen mit aktiven H-Atomen kann bei Temperaturen von 90 bis 260°C vorgenommen werden. Für großtechnische Verfahren sind dabei Temperaturen von 150 bis 210°C bevorzugt. Der Reaktionsdruck beträgt vorzugsweise 1 bis 8 bar. Es ist jedoch auch möglich, die Alkoxylierungsreaktion bei Drucken außerhalb dieses Bereiches durchzuführen, beispielsweise bei 0,7 bar oder bei Drucken oberhalb von 8 bar.

Sollen die organischen Verbindungen mit aktiven H-Atomen mit Ethylenoxid und Propylenoxid umgesetzt werden, so kann die Alkoxylierung sowohl als Blockpolymerisation als auch als Randompolymerisation durchgeführt werden.

Erfindungsgemäß werden die Erdalkalisalze von Ethercarbonsäuren der allgemeinen Formel I als Katalysatoren in einer Menge von 0,1 bis 2 Gew.-%, bezogen auf das Endprodukt der Ethoxylierung bzw. Propoxylierung, eingesetzt.

Die Erfindung wird im folgenden anhand von bevorzugten Ausführungsbeispielen näher erläutert.

A. Allgemeine Herstellungsvorschrift für Calcium-, Strontium- und Bariumsalze von Ethercarbonsäuren der allgemeinen Formel I.

Die Ethercarbonsäuren wurden in einem Gemisch von Wasser, Ethanol und Isopropanol (3 : 2 : 1) gelöst bzw. aufgeschlämmt und bei 90°C mit äquimolaren Mengen Calcium-, Strontium- bzw. Bariumacetat (Menge berechnet über die Säurezahl) umgesetzt. Die freiwerdende Essigsäure wurde mit dem Lösemittel abdestilliert.

Es wurden die in der folgenden Tabelle I zusammengefaßten Katalysatoren hergestellt.

## Tabelle I

Hergestellte Katalysatoren der allgemeinen Formel I

| Katalysator Nr. | R | m | n | x | M |
|---|---|---|---|---|---|
| 1 | $C_{12/14}$ | 2 | 3,8 | 2 | Ba |
| 2 | $C_{12/18}$ | 2 | 9 | 2 | Ba |
| 3 | $CH_2COO^-$ | 2 | 3 | 1 | Ca |
| 4 | $C_{12}$ | 2 | 4 | 2 | Ca |
| 5 | $C_8$ | 2 | 3 | 2 | Ca |
| 6 | $C_8$ | 2 | 5 | 2 | Ca |
| 7 | $C_8$ | 2 | 5 | 2 | Ba |
| 8 | $C_8$ | 2 | 5 | 2 | Sr |

## Tabelle II

Ethoxylierung eines handelsüblichen Laurylalkohols (Lorol$^R$ $C_{12}$) unter erfindungsgemäßer Verwendung von Katalysatoren der Formel I

| Kat.-Nr. | Kat.-% | t (h) | Q | FFA (%) | OH-Zahl ist | OH-Zahl soll |
|---|---|---|---|---|---|---|
| 1 | 0,5 | 6,5 | 1.279 | 4,6 | 131,2 | 125,2 |
| 2 | 0,5 | 7,5 | 850 | 4,4 | 128,1 | 122,2 |
| 3 | 0,5 | 6,0 | 1.100 | 4,9 | 136 | 126,3 |
| 4 | 0,5 | 7,0 | | | 116,6 | 125,2 |
| 5 | 0,5 | 11,0 | 1.112 | 5,0 | 134,1 | 123,7 |
| 6 | 0,5 | 6,5 | 1.086 | 5,9 | 137,7 | 128,9 |
| 7 | 0,5 | 3 | 1.006 | 5,3 | 131,8 | 130,0 |
| 8 | 0,5 | 3 | 983 | 4,4 | 135,8 | 127,0 |

B. Ethoxylierung mit den erfindungsgemäß zu verwendenden Katalysatoren der allgemeinen Formel I.

Der Katalysator wurde in der zu ethoxylierenden Substanz gelöst. Die Lösung wurde in einen für die Alkoxylierung geeigneten Autoklaven überführt. Es wurde mit Stickstoff gespült und 30 Minuten lang bei einer Temperatur von 100°C evakuiert. Anschließend wurde die Temperatur auf 180°C gesteigert; die gewünschte Menge Ethylenoxid wurde bei einem Druck von 5 bar aufgedrückt. Nach Beendigung der Reaktion ließ man 30 Minuten nachreagieren.

Die nach dieser Arbeitsvorschrift mit den erfindungsgemäß eingesetzten Katalysatoren bei der Ethoxylierung eines handelsüblichen Laurylalkohols (Lorol® $C_{12}$) erhaltenen Ergebnisse sind in der Tabelle II zusammengefaßt. Es bedeuten

Q = der oben genannte Q-Wert,
OH-Zahl, ist/soll = OH-Zahlen der als Endprodukt erhaltenen polyethoxylierten Laurylalkohole
Kat.-% = Katalysatorkonzentration, bezogen auf das Endprodukt
FFA = Gehalt des Endproduktes an freien Fettalkoholen in Flächen-% (GC-Analyse)
t = Reaktionszeit der Polyethoxylierung.

In Tabelle III sind die Ergebnisse von Vergleichsversu chen mit bekannten Katalysatoren zusammengefaßt. Es wird ebenfalls ein handelsüblicher Laurylalkohol polyethoxyliert. Der Vergleich zeigt, daß die Verbindungen der Erfindung bessere Homologenverteilungen (größere Q-Werte) als Kaliumhydroxid, Natriummethylat und Calciumhydroxid ergeben. Sie liefern weiterhin ähnlich gute Ergebnisse wie Bariumhydroxid, Calciumoleat und Calciumstearat, sind im Gegensatz zu diesen jedoch in dem Reaktionsgemisch löslich. Calciumethylat, das ebenfalls ähnlich gute Ergebnisse liefert, ist wesentlich schwerer herzustellen und nur unter Schwierigkeiten handhabbar.

Tabelle III

Ethoxylierung eines handelsüblichen Laurylalkohols (Lorol[R] $C_{12}$) unter Verwendung bekannter Katalysatoren

| Katalysator | Kat-% | Rkt. Zeit (H) | Q | OH-Zahl ist | OH-Zahl soll | FFA (%) |
|---|---|---|---|---|---|---|
| KOH | 0,5 | 3,5 | 611 | 125,1 | 125,2 | 7,5 |
| $NaOCH_3$ | 0,5 | 4,0 | 595 | 128,8 | 126,8 | 9,9 |
| $Ca(OH)_2$ | 0,5 | 11,0 | 695 | 136,8 | 126,3 | 9,4 |
| $Ba(OH)_2$ | 1,0 | 4,0 | 1225 | 129,3 | 128,4 | 3,8 |
| Ca-Oleat | 0,5 | 10,5 | 1272 | 126,7 | 128,4 | 5,1 |
| Ca-Stearat | 1,0 | 7,5 | 1251 | 132,8 | 129,5 | 5,0 |
| Ca-Ethylat | 0,5 | 8,0 | 1316 | 130,2 | 125,7 | 4,5 |

## Patentansprüche

1. Verwendung von Erdalkalisalzen von Ethercarbonsäuren der allgemeinen Formel I
$[RO\text{-}(C_mH_{2m}O)_n\text{-}CH_2\text{-}COO^-]_x\ M^{2+}$(I)
in der
R ein Rest aus der von $C_1$ bis $C_{22}$-Alkyl, $C_3$ bis $C_{22}$-Alkenyl, Phenyl, Alkylphenyl mit 1 bis 3 $C_1$ bis $C_{15}$-Alkylresten, Benzyl und Phenylethyl gebildeten Gruppe,
M ein Erdalkalimetall aus der von Ca, Sr und Ba gebildeten Gruppe,
m die Zahl 2 und/oder 3 und
n die Zahl 1 bis 20 und
x die Zahl 2
oder
R eine Gruppe der Formel
$-CH_2\text{-}COO^-$
x die Zahl 1
und
M, m und n wie oben definiert sind,
als Katalysatoren für die Ethoxylierung und/oder Propoxylierung von organischen Verbindungen mit aktiven H-Atomen.

2. Verwendung von Erdalkalisalzen von Ethercarbonsäuren der allgemeinen Formel I nach Anspruch 1, in der
m die Zahl 2 ist sowie R, M, n und x wie oben definiert sind.

3. Verwendung von Erdalkalisalzen von Ethercarbonsäuren der allgemeinen Formel I nach Anspruch 1, in der R ein Rest aus der von $C_1$- bis $C_8$-Alkyl gebildeten Gruppe ist.

4. Verwendung von Erdalkalisalzen von Ethercarbonsäuren der allgemeinen Formel I nach Anspruch 1, in der n eine Zahl von 1 bis 12 bedeutet.

5. Verwendung nach einem der Ansprüche 1 bis 4, dadurch gekennzeichnet, daß man die Katalysatoren in einer Menge von 0,1 bis 2 Gew.-%, bezogen auf das Endprodukt der Ethoxylierung bzw. Propoxylierung, einsetzt.

## Claims

1. The use of alkaline earth metal salts of ether carboxylic acids corresponding to general formula I
$[RO\text{-}(C_mH_{2m}O)_n\text{-}CH_2\text{-}COO^-]_x\ M^{2+}$ (I)
in which
R is a radical from the group consisting of $C_{1-22}$
alkyl, $C_{3-22}$
alkenyl, phenyl, alkyl phenyl containing 1 to 3 $C_{1-15}$
alkyl radicals, benzyl and phenyl ethyl,
M is an alkaline earth metal from the group consisting of Ca, Sr and Ba,
m is the number 2 and/or 3 and
n is a number of 1 to 20 and
x is the number 2
or
R represents a group corresponding to the formula
$-CH_2\text{-}COO^-$
x is the number 1 and
M, m and n are as defined above,
as catalysts for the ethoxylation or propoxylation of organic compounds containing active hydrogen atoms.

2. The use of alkaline earth metal salts of ether carboxylic acids corresponding to general formula I in claim 1 in which m is the number 2 and R, M, n and x are as defined above.

3. The use of alkaline earth metal salts of ether carboxylic acids corrsponding to general formula I in claim 1, in which R is a radical from the group consisting of $C_{1-8}$
alkyl radicals.

4. The use of alkaline earth metal salts of ether carboxylic acids corresponding to general formula I in claim 1, in which n is a number of 1 to 12.

5. The use claimed in any of claims 1 to 4, characterized in that the catalysts are used in a quantity of 0.1 to 2% by weight, based on the ethoxylated end product.

**Revendications**

1. Mise en oeuvre de sels de métaux alcalino-terreux d'acides éthercarboxyliques de formule générale I

$[RO-(C_mH_{2m}O)_n-CH_2-COO^-]_x M^{2+}$ (I)

dans laquelle

R représente un radical faisant partie du groupe constitué de: alkyle de $C_1$ à $C_{22}$, alcényle de $C_3$ à $C_{22}$, phényle, alkylphényle comportant 1 à 3 radicaux alkyle de $C_1$ à $C_{15}$, benzyle et phényléthyle,

M est un métal alcalino-terreux appartenant au groupe formé par le calcium, le strontium et le baryum,

m est égal à 2 et/ou à 3,

n est un nombre compris dans la plage de 1 à 20 et

x est égal à 2

ou

R est un groupement de formule

$-CH_2-COO^-$

x est égal à 1

et

M, m et n possèdent la valeur indiquée ci-dessus,

comme catalyseurs pour l'éthoxylation et/ou la propoxylation de composés organiques à atomes d'hydrogène actifs.

2. Mise en oeuvre de sels de métaux alcalino-terreux d'acides éthercarboxyliques (de formule générale I selon la revendication 1, dans laquelle m est égal à 2, tandis que R, M, n et x possèdent la valeur indiquée ci-dessus.

3. Mise en oeuvre de sels de métaux alcalino-terreux d'acides éthercarboxyliques de formule générale I selon la revendication 1, dans laquelle R est un radical du groupe formé par les groupements alkyle de $C_1$ à $C_8$.

4. Mise en oeuvre de sels de métaux alcalino-terreux d'acides éthercarboxyliques de formule générale I selon la revendication 1, dans laquelle n représente un nombre de 1 à 12.

5. Mise en oeuvre selon l'une des revendications 1 à 4, caractérisée en ce que l'on met en oeuvre les catalyseurs dans une proportion allant de 0,1 à 2% en poids, par rapport au produit final de l'éthoxylation ou de la propoxylation.